Europäisches Patentamt

(19) European Patent Office

Office européen des brevets

(11) Publication number : **0 360 556 B1**

# (12) EUROPEAN PATENT SPECIFICATION

(45) Date of publication of patent specification :
**21.04.93 Bulletin 93/16**

(51) Int. Cl.⁵ : **A61K 35/78**

(21) Application number : **89309500.0**

(22) Date of filing : **19.09.89**

(54) New extracts of ginkgo biloba and their methods of preparation.

(30) Priority : **20.09.88 GB 8822004**

(43) Date of publication of application :
**28.03.90 Bulletin 90/13**

(45) Publication of the grant of the patent :
**21.04.93 Bulletin 93/16**

(84) Designated Contracting States :
**AT BE CH DE ES FR GB GR IT LI LU NL SE**

(56) References cited :
**EP-A- 0 086 315**

(73) Proprietor : **INDENA S.p.A.**
**Via Ripamonti, 99**
**I-20141 Milano (IT)**

(72) Inventor : **Bombardelli, Ezio**
**Via Val Di Sole 22**
**Milano (IT)**
Inventor : **Mustich, Giuseppe**
**Via Washington 57**
**Milano (IT)**
Inventor : **Bertani, Marco**
**Via A. Vespucci 18**
**Abbiategrasso (IT)**

(74) Representative : **Ritter, Stephen David et al**
**Mathys & Squire 10 Fleet Street**
**London EC4Y 1AY (GB)**

## Description

The present invention relates to a process for producing extracts of Ginkgo biloba leaves. The invention particularly relates to a procedure which can lead to extracts having chemical compositions and biological activities which correspond closely to those described in the literature for extracts of Ginkgo biloba, since the time when derivatives of the plant were introduced into the European market for therapeutic use. The process of the invention enables the production of new extracts which find application in both the therapeutic and the cosmetic field.

Methods of preparing active extracts of Ginkgo biloba leaves have been described in DE 1767098 and DE 2117429 (Dr. Willman Schwabe) and in JP 1167714. For some extracts prepared in accordance with the process described in these patents, activities are claimed which cannot readily be ascribed to a single class of compounds. This is in contrast to what can be inferred from the simple chemical characterisation of the extracts quoted in these patents.

In fact, in these patents, reference is made in a very superficial manner to the classes of active principles responsible for the multiplicity of actions which are claimed. For example, DE 2117429, describes an improvement of the procedure of DE 1767098, by which purification is effected to permit injectability of the resulting preparation. According to this process, catechic polymeric derivatives of a tannic nature normally not compatible with the blood are apparently eliminated leaving the remaining portion unchanged.

Subsequent research has made it clear that some constituents present in the extracts obtained by the processes initially described have special activities. For example, in BE 902874 and ZA 8848369 there are described activities relating to individual components, such as the ginkgolides and bilobalide, which were normally present in the known extracts previously employed in therapy (see e.g. the work of S.S. Chatteyee, Arztezeitschrift für Naturheilverfahren 22, 595-604, 1981).

The known extracts prepared from Ginkgo biloba, which have been used in medicine since 1965 for the therapy of cerebral disturbances and pathologies correlated with changes in the peripheral circulation, contain (at least as far as it is possible to determine with the analytical methods currently available) flavonic glucosides as principal components. Among these, the most important have proved to be 5,7,3,4-tetrahydroxyflavone-3D-α-rhamnopyranosyl-4-D-β-O-(6‴-transcoumaroyl)-glucopyranoside and 5,7,3,4',5'pentahydroxyflavan-3D-α-rhamnopyranosyl-4-β-O-(6‴ coumaroyl)glucopyranoside.

As is apparent from the work described by Chatteyee (supra), ginkgolides and bilobalide are present in the known extracts. Also present in these extracts are other flavonoids such as quercetin -3-rhamnoside and kaempferol-3-rhamnoside, luteolin-7-glucoside and derivatives of isorhamnetin and of quercetin, such as rutin and quercetin-3-glucoside.

Analyses carried out by us on medicinal specialities on the market in various European countries have supplied data which are in perfect accord both with the work published by Chatteyee (supra) and with the more recent work reported in La Presse Médicale 15, 1455-7, 1986, and in "Flavonoids and Bioflavonoids" Eds. L. Farkas, Elsevier 1986, page 351.

Among the flavones, it has been verified that the principal components are in effect the coumaroyl derivatives respectively of 3-rhamnoglucoside of quercetin and of caempferol.

This last compound is today considered to be the active constituent (or the most important active constituent) of the flavonoidic fraction, as reported in the work of Braquet et al. ("Plant Flavonoids in Biology and Medicine" Strasbourg 1987), while of lesser importance in terms of biological activity are the other constituents having a flavonic skeleton.

As a result of analyses carried out by HPLC and by gas chromatography/mass spectrometry, it has become apparent that in the extracts used up to today in therapy, significant amounts of ginkgolides and bilobalide are present, as well as substances which are minor components, but which are important for the purposes of bioavailability, such as p-hydroxybenzoic acid, kynurenic acid and hydroxykynurenic acid, etc (La Presse Médicale, supra). The extracts have moreover been characterised by us as regards the known polyphenolic portion through the "procyanidolic index", which supplies an indirect value of the content of catechic derivatives (after acid hydrolysis), i.e. the quantity of anthocyanidines formed by acid catalysed decomposition of oligomeric condensed polyphenols. This characterisation indicates the type of oligomer present on the basis of the catechic and anthocyanidinic monomers which are liberated. In these analyses, in fact, catechin, gallocatechin, cyanidin and delphinidin have been determined, and provide a pointer to the catechic nature of the oligomeric polyphenolic fraction.

Following these studies, and this constitutes one of the objects of this invention, a new industrial process has been developed for the preparation of Ginkgo biloba extracts. The new procedure allows the production of extracts which can be identical (so far as can be reasonably established for a plant derivative) to the products employed today in therapy and which contain the various classes of known substances in Ginkgo biloba, but enables drawbacks of known extraction processes to be avoided.

The process of the invention further allows the production of two novel extracts. One is constituted by a dimeric flavonoids fraction, and provides a new active product useful in the therapy of disturbances of the cerebral and peripheral circulation, in disorders due to platelet aggregation and in those morbid manifestations in which antiphosphodiesterasic activity is useful. The second comprises an extract constituted by the total polyphenolic fraction and which is useful, above all, for the treatment of superficial vasculopathies and for topical treatments of changes in the microcirculation.

This last extract can be produced in a form which contains all the free flavonoidic substances and their glucosidated derivatives present in Ginkgo biloba, as well as the proanthocyanidinic oligomeric fraction characteristic of the plant. This last extract preferably does not contain lipophilic substances which are poorly soluble in water.

According to the scientific and patent literature, the processes hereinbefore described involve extraction with pure aliphatic alcohols or ketones or with aqueous mixtures of such alcohols or ketones which are miscible with water, employing predetermined amounts of solvents. The following stages involved direct counter-extraction of the extracts with halogenated hydrocarbons to remove the lipophilic substances. Finally, after saturating the aqueous solution with ammonium sulphate with or without treatment of the solution with lead (or other heavy metals) salts or with polyamides (when it was intended to reduce the tanninic part) the active principles were extracted with methyl ethyl ketone. The ketonic organic phase was dehydrated, concentrated to a low volume and, after treatment with ethanol, evaporated to dryness. These methods had the fundamental disadvantage of using large volumes of different solvents which are miscible with one another, very large quantities of salts for facilitating the extraction and the separation of the phases and, in some situations, polluting lead salts.

We have now developed a procedure for obtaining purified extracts of Ginkgo biloba which avoids or reduces drawbacks of prior art processes. Furthermore, the process of the invention enables extracts to be obtained which contain the active components of the plant in proportions which can be selected, if desired, to correspond to those present in the plant itself or in known extracts.

According to the present invention, there is provided a process for producing a purified extract comprising a plurality of the active components of Ginkgo biloba leaves, which comprises subjecting a crude or partially purified extract of Ginkgo biloba leaves to a plurality of solvent extraction procedures, characterised in that at least one of said solvent extraction procedures employs a solvent comprising a $C_{6-8}$ aromatic hydrocarbon, preferably toluene and a $C_{3-6}$ lower alkanol, preferably n-butanol.

The use of a solvent comprising an aromatic hydrocarbon and a lower alkanol offers a number of significant advantages in the production of Ginkgo biloba extracts, including the following:

(1) it has been found that such solvents are particularly effective in extracting a large number of the desired active components,

(2) the solvents, particularly toluene and butanol, are miscible and it is possible to produce a range of solvents having differing affinities of substances of different polarities. Thus relatively lipophilic (i.e. less polar or hydrophobic) substances may be extracted using solvents containing a relatively high proportion of aromatic hydrocarbon, while hydrophilic (i.e. more polar) substances may be extracted using solvents containing a relatively high proportion of lower alkanol.

(3) extraction procedures may be devised in which the number of different organic solvents used is low (e.g. two or three). This gives rise to significant cost savings and increases the ease with which valuable solvents may be recycled.

(4) the use of heavy metal salts may be avoided.

(5) the selected solvents present less toxicity hazards than chlorinated hydrocarbon solvents used in defatting steps of prior art processes.

By way of example of advantages (2) and (5), in accordance with one preferred method of operation according to the invention, a solvent comprising a major proportion of toluene and a minor proportion of n-butanol may be used to extract fatty materials from an extract of Ginkgo biloba leaves. Preferably the volume ratio of toluene: butanol is greater than 6:1, most preferably about 9:1.

By way of example of advantages (1) and (2), in accordance with another preferred method of operation according to the invention, a solvent comprising a minor proportion of toluene and a major proportion of n-butanol may be used to extract desired active components from an extract of Ginkgo biloba leaves. Preferably the volume ratio of toluene:butanol is less than 1:2 and most preferably is about 1:4.

The crude or partially purified extract used in the process of the invention may be obtained by any convenient means.

According to a preferred method of preparing said crude or partially purified extract, a starting material formed from finely ground, still green, machine-dried leaves, is extracted to exhaustion with an aqueous solvent comprising a mixture of acetone and water or a mixture of methanol and/or ethanol and water. The concentrations of the organic component expressed as a percentage of the total volume of solvent employed preferably comprises between 45 and 99%. The extraction temperature is preferably between 15 and 70°C.

The resulting crude or partially purified aqueous extracts can then be treated directly, i.e. without a prior concentration step, in order to remove inactive lipophilic substances. For example they may be extracted directly with n-hexane or with n-heptane. In accordance with a preferred embodiment of the invention, the crude or partially purified extracts may be contacted with an aromatic hydrocarbon/lower alkanol mixture to remove inactive lipophilic substances such as chlorophyll, ginkgolic acids, polyprenols, aliphatic alcohols and free and esterified sterols. The hexane or the aromatic hydrocarbon/lower alkanol mixture used as solvents do not extract, for example, ginkgolides and bilobalides (which are also lipophilic substances) but whose presence is desired in the final product.

Alternatively, the water-acetone, water-methanol or water-ethanol extracts may be concentrated to a water solution having a volume equal to double the weight of the drug. In this case, the concentrate is diluted with alcohol (ethanol or methanol) or with acetone so as to re-establish an appropriate ratio of water/organic solvent prior to extraction of lipophilic substances.

The defatted solution may then be concentrated to a volume equal to the weight of the drug and then the concentrate may be kept in a refrigerator for 24 hours at a temperature of about 2°C and then centrifuged. The semi-crystalline precipitate which is separated by centrifuging comprises a mixture of dimeric flavonoids (sciadopitisin, ginkgonetin, isoginkgonetin, bilobetin, amentoflavone) which can be employed as such or after recrystallization for formulations for therapeutic or cosmetic use.

These mixtures readily extract substantially all the active principles present, but extract the condensed polyphenolic substances such as the tannins with extreme difficulty, for which reason the solvents may be regarded as being selective. Naturally, these solvent mixtures do not extract free sugars, polysaccharides, common organic and inorganic salts and proteins and their derivatives.

After careful counterwashing with water, the toluene-butanol phase may be concentrated under vacuum to a paste-like consistency at a temperature not higher than 40°C and taken up with water or a water-alcohol mixture in order to remove the residual traces of toluene and butanol and dried.

The aqueous solution, which has been defatted and still contains a proportion of the dimeric flavones, can be passed over absorption resins such as Amberlite XAD4, XAD26 or Duolite S-761 these resins readily absorb many substances and have a marked activity for those of a phenolic nature; the same substances can be re-eluted from the resin with pure alcohols or ketones or alcohols or ketones in suitable mixtures with water.

(Duolite S-761 is a hydroxylic phenol-formaldehyde absorption resin with a granulometry of 0.3 to 1.2 mm.)

Thus according to a preferred manner of operation in accordance with the invention an aqueous phase recovered during the purification procedure is contacted with an absorption resin and absorbed flavonoids are eluted with an organic solvent.

The absorption resin preferably comprises an aromatic polymer, for example a hydroxyl group containing phenol-formaldehyde resin. The organic solvent is preferably a lower ($C_{1-4}$) alkanol or a water-miscible ketone, either of which may be used in pure form or in admixture with water.

In this case, after absorption and re-elution of the active principles, it is possible to obtain an extract rich in the flavonoid component which lends itself, like the others, to being incorporated in the most common pharmaceutical formulations and, in this specific case, also cosmetic formulations.

The Examples given hereinafter illustrate the more important features of the invention without, however, being limitative.

## Example I

100 kg of finely ground, dried Ginkgo biloba leaves are extracted under agitation 4 times with 400 l of a mixture of acetone and water in a ratio of 60:40 (w/w) at a temperature around 50°C.

The combined water-acetone extracts are extracted continuously in countercurrent with 500 l of n-hexane. In the final stage, the n-hexane solution appears practically colourless. The defatted water-acetone solution is concentrated under vacuum to 200 l at 40°C; the aqueous concentrate is cooled to 2°C over night and is then centrifuged. After washing with water and drying, 400 g of a product constituted predominantly by dimeric flavonoids derived from the apigenin are obtained.

The aqueous solution filtered from the dimeric flavonoid fraction is subjected to countercurrent extraction with about 400 l of a mixture of n-butanol and toluene in a volume ratio of 8:2.

The organic phase is washed carefully with demineralised water and is concentrated to a paste under vacuum at a temperature not higher than 40°C. It is taken up with water and dried. The residue is taken up in 50 l of aqueous ethanol at 70%. After filtration, the water-ethanol solution is concentrated to 25 l and the concentrate is atomized.

1.98 kg of yellow-beige extract having the following characteristics are obtained:

| | |
|---|---|
| Content of ginkgoflavoneglucosides | 24% |
| Procyanidolic index | 9% |
| Sulphuric ash | 0.1% |
| Content of ginkgolides | 3.6% |
| Content of bilobalide | 3.1% |

Example II

100 kg of finely ground Ginkgo biloba leaves are extracted four times with 400 l of an aqueous methanol mixture in a ratio of 1:1 (v/v) at 35°C. The combined extracts are concentrated to 100 l under vacuum and at a temperature not higher than 40°C. The concentrate is diluted with 100 l of methanol and the whole is filtered. The filtered solution is extracted three times with 100 l of an 9:2 toluene-butanol mixture. The organic solution is counterwashed with 50 l of a 1:1 methanol-water mixture. The combined water-methanol phases are concentrated to water and, after filtration, are extracted with 2:8 toluene-n-butanol mixture. After counterwashing with water, the organic phase is dehydrated over sodium sulphate, filtered and concentrated to dryness; the residue is solubilized in 10 volumes of a 2:8 ethanol-water mixture, filtered and atomized. 2.2 kg of product similar to that of Example I are obtained.

Example III

100 kg of finely ground Ginkgo biloba leaves are extracted four times with 400 l of 60% aqueous methanol (w/w) to exhaustion of active principles.

The combined extracts are concentrated to 350 l at a temperature not higher than 40°C with addition of acetone and adjustment of the acetone concentration to about 50%.

The so obtained solution is filtered so as to eliminate any lipophyl resinous impurity and extracted two times with 100 l of a 9:1 toluene-butanol mixture.

The hydroacetone phase is concentrated under vacuum until complete elimination of the organic solvent and chromatographed on a column packed with 100 l of Duolite S-761 absorption resin.

After absorption of the solution, continue washing the column with about 300 l of purified water so as to remove salts, sugars and unwanted hydrophilic substances. Elute the column with 90% aqueous methanol and continue washing until a colourless solvent is obtained. The eluate is concentrated to dryness: 2.5 kg of product is obtained. The solubility characteristics make it fit for pharmaceutical as well as cosmetic application.

It will be appreciated that the present invention provides a novel process for preparing extracts of Ginkgo biloba with a standardized content of active principles which may be similar to those presently available in therapy for the treatment of vascular disturbances of the peripheral and cerebral circulation. The invention also provides processes for isolating another two extracts, one constituted by the flavonoid fraction represented solely by the dimeric flavones characteristic of this plant and having activity on the peripheral circulation and platelet aggregation, showing a marked antiphosphodiesterasic action, while the second is constituted by the total polyphenolic fraction of the plant which can find use in both the dermatological and the cosmetic field as an anti-inflammatory and vasokinetic agent.

The terms "Amberlite" and "Duolite" as used herein are trademarks.

**Claims**

1.    A process for producing a purified extract comprising a plurality of the active components of Ginkgo biloba leaves, which comprises subjecting a crude or partially purified extract of Ginkgo biloba leaves to a plurality of solvent extraction procedures, characterised in that at least one of said solvent extraction procedures employs a solvent comprising a $C_{6-8}$ aromatic hydrocarbon and a $C_{3-6}$ lower alkanol.

2. A process according to Claim 1 in which the aromatic hydrocarbon is toluene.

3. A process according to Claim 1 or Claim 2 in which the lower alkanol is n-butanol.

4. A process according to any preceding claim wherein an extraction procedure which employs a solvent comprising a $C_{6-8}$ aromatic hydrocarbon and a $C_{3-6}$ lower alkanol is used to extract fatty materials from an extract of Ginkgo biloba leaves, said solvent containing a major proportion of the $C_{6-8}$ aromatic hydrocarbon.

5. A process according to Claim 4 in which a solvent comprising toluene and n-butanol in a volume ratio of toluene:butanol greater than 6:1 is used to extract fatty materials from an extract of Ginkgo biloba leaves.

6. A process according to Claim 4 wherein the volume ratio of toluene:butanol is about 9:1.

7. A process according to any preceding claim wherein an extraction procedure which employs a solvent comprising a $C_{6-8}$ aromatic hydrocarbon and a $C_{3-6}$ lower alkanol is used to extract desired active components from an extract of Ginkgo biloba leaves, said solvent containing a minor proportion of the $C_{6-8}$ aromatic hydrocarbon.

8. A process according to claim 7 in which a solvent comprising toluene and n-butanol in a volume ratio of toluene:butanol less than 1:2 is used to extract desired active components from an extract of Ginkgo biloba leaves.

9. A process according to claim 8 wherein the volume ratio of toluene:butanol is in the range 1:2 to 1:5.

10. A process according to Claim 9 wherein the volume ratio of toluene:butanol is about 1:4.

11. A process according to any preceding claim wherein an aqueous phase is recovered as a product of said solvent extraction procedures, and dimeric flavonoids are recovered from said aqueous phase.

12. A process according to any of claims 1 to 10 wherein an aqueous phase recovered during the purification procedure is contacted with an absorption resin and absorbed flavonoids are eluted with an organic solvent.

13. A process according to Claim 12 wherein the absorption resin comprises an aromatic polymer.

14. A process according to claim 12 or claim 13 wherein the absorption resin comprises a hydroxy group-containing phenol-formaldehyde resin.

15. An extract of Ginkgo biloba characterised by the presence of flavonoidic substances, their glucosidated derivatives, ginkgolides, bilobalide and proanthocyanidinic oligomeric substances and the substantial absence of inactive lipophilic substances which can be extracted by n-hexane, n-heptane or a solvent comprising a $C_{3-6}$ lower alkanol and a major proportion of a $C_{6-8}$ aromatic hydrocarbon.

16. An extract as claimed in claim 15 which is obtainable by a process as claimed in any of claims 1 to 14.

17. An extract as claimed in any of claims 15 to 16 having a composition substantially as follows:

| | |
|---|---|
| Content of ginkgoflavoneglucosides | 24% |
| Procyanidolic index | 9% |
| Content of ginkgolides | 3.6% |
| Content of bilobalide | 3.1% |

**Patentansprüche**

1.  Verfahren zur Herstellung eines gereinigten, eine Mehrzahl der aktiven Komponenten von Ginkgo biloba-Blättern umfassenden Extraktes, wobei das Verfahren den Schritt umfaßt, daß man einen rohen oder teilweise gereinigten Extrakt von Ginkgo biloba-Blättern einer Mehrzahl von Lösungsmittelextraktions-Verfahrensschritten unterwirft, dadurch gekennzeichnet, daß wenigstens einer der Lösungsmittelextraktions-Verfahrensschritte ein Lösungsmittel einsetzt, das einen aromatischen $C_{6-8}$-Kohlenwasserstoff und ein niederes $C_{3-6}$-Alkanol umfaßt.

2.  Verfahren nach Anspruch 1, worin der aromatische Kohlenwasserstoff Toluol ist.

3.  Verfahren nach Anspruch 1 oder Anspruch 2, worin das niedere Alkanol n-Butanol ist.

4.  Verfahren nach irgendeinem der vorangehenden Ansprüche, worin ein Extraktionsverfahren eingesetzt wird, in dem ein Lösungsmittel verwendet wird, das einen aromatischen $C_{6-8}$-Kohlenwasserstoff und ein niederes $C_{3-6}$-Alkanol umfaßt, um Fettstoffe aus einem Extrakt von Ginkgo biloba-Blättern zu extrahieren, wobei das Lösungsmittel einen größeren Anteil des aromatischen $C_{6-8}$-Kohlenwasserstoffs enthält.

5.  Verfahren nach Anspruch 4, worin ein Lösungsmittel verwendet wird, das Toluol und n-Butanol in einem Volumenverhältnis Toluol : Butanol größer als 6 : 1 umfaßt, um Fettstoffe aus einem Extrakt von Ginkgo biloba-Blättern zu extrahieren.

6.  Verfahren nach Anspruch 4, worin das Volumenverhältnis Toluol : Butanol etwa 9 : 1 ist.

7.  Verfahren nach irgendeinem der vorangehenden Ansprüche, worin ein Extraktionsverfahren verwendet wird, in dem ein Lösungsmittel eingesetzt wird, das einen aromatischen $C_{6-8}$-Kohlenwasserstoff und ein niederes $C_{3-6}$-Alkanol umfaßt, um gewünschte aktive Komponenten aus einem Extrakt von Ginkgo biloba-Blättern zu extrahieren, wobei das Lösungsmittel zum kleineren Anteil den aromatischen $C_{6-8}$-Kohlenwasserstoff enthält.

8.  Verfahren nach Ansprüch 7, worin ein Lösungsmittel verwendet wird, das Toluol und n-Butanol in einem Volumenverhältnis von Toluol : Butanol Heiner als 1 : 2 umfaßt, um gewünschte aktive Komponenten aus einem Extrakt von Ginkgo biloba-Blättern zu extrahieren.

9.  Verfahren nach Anspruch 8, worin das Volumenverhältnis von Toluol : Butanol im Bereich von 1 : 2 bis 1 : 5 liegt.

10. Verfahren nach Anspruch 9, worin das Volumenverhältnis von Toluol : Butanol etwa 1 : 4 ist.

11. Verfahren nach irgendeinem der vorangehenden Ansprüche, worin eine wäßrige Phase als Produkt der Lösungsmittelextraktionsschritte gewonnen wird und dimere Flavonoide aus der wäßrigen Phase gewonnen werden.

12. Verfahren nach irgendeinem der Ansprüche 1 bis 10, worin eine während des Reinigungsverfahrens gewonnene wäßrige Phase mit einem Absorptionsharz in Kontakt gebracht wird und absorbierte Flavonoide mit einem organischen Lösungsmittel eluiert werden.

13. Verfahren nach Anspruch 12, worin das Absorptionsharz ein aromatisches Polymer umfaßt.

14. Verfahren nach Anspruch 12 oder Anspruch 13, worin das Absorptionsharz ein Hydroxygruppen enthaltendes Phenol-Formaldehyd-Harz umfaßt.

15. Extrakt von Ginkgo biloba, gekennzeichnet durch die Anwesenheit flavonoider Substanzen, ihrer glukosidierten Derivate, Ginkgolide, Bilobalid und proanthocyanidiner oligomerer Substanzen und im wesentlichen die Abwesenheit inaktiver lipophiler Substanzen, die mit n-Hexan, n-Heptan oder einem Lösungsmittel extrahiert werden können, das ein niederes $C_{3-6}$-Alkanol und einen größeren Anteil an einem aromatischen $C_{6-8}$-Kohlenwasserstoff umfaßt.

16. Extrakt nach Anspruch 15, der durch ein Verfahren erhältlich ist, wie es in irgendeinem der Ansprüche 1

EP 0 360 556 B1

bis 14 beansprucht ist.

17. Extrakt nach irgendeinem der Ansprüche 15 bis 16 mit einer Zusammensetzung, die im wesentlichen die folgende ist:

| | | |
|---|---|---|
| Gehalt an Ginkgoflavonglukosiden | 24 | % |
| Procyanidolin-Index | 9 | % |
| Gehalt an Ginkgoliden | 3,6 | % |
| Gehalt an Bilobalid | 3,1 | %. |

**Revendications**

1. Procédé de préparation d'un extrait purifié comprenant plusieurs constituants actifs de feuilles de Ginkgo biloba, qui comprend le fait de soumettre un extrait brut ou partiellement purifié de feuilles de Ginkgo biloba à plusieurs opérations d'extraction par les solvants, caractérisé en ce qu'au moins une de ces opérations d'extraction par les solvants utilise un solvant comprenant un hydrocarbure aromatique en $C_6$ à $C_8$ et un alcanol inférieur en $C_3$ à $C_6$.

2. Procédé selon la revendication 1, dans lequel l'hydrocarbure aromatique est le toluène.

3. Procédé selon les revendications 1 ou 2, dans lequel l'alcanol inférieur est le n-butanol.

4. Procédé selon l'une quelconque des revendications précédentes, dans lequel on utilise une opération d'extraction qui utilise un solvant comprenant un hydrocarbure aromatique en $C_6$ à $C_8$, et un alcanol inférieur en $C_3$ à $C_6$, pour extraire les matières grasses d'un extrait de feuilles de Ginkgo biloba, ce solvant contenant une proportion prédominante des hydrocarbures aromatiques en $C_6$ à $C_8$.

5. Procédé selon la revendication 4, dans lequel on utilise un solvant comprenant du toluène et du n-butanol dans un rapport volumique toluène : butanol supérieur à 6 : 1 pour extraire les matières grasses d'un extrait de feuilles de Ginkgo biloba.

6. Procédé selon la revendication 4, dans lequel le rapport volumique toluène : butanol est d'environ 9 : 1.

7. Procédé selon l'une quelconque des revendications précédentes, dans lequel on utilise une technique d'extraction utilisant un solvant comprenant un hydrocarbure aromatique en $C_6$ à $C_8$ et un alcanol inférieur en $C_3$ à $C_6$ pour extraire les constituants actifs désirés d'un extrait de feuilles de Ginkgo biloba, ce solvant contenant une proportion mineure des hydrocarbures aromatiques en $C_6$ à $C_8$.

8. Procédé selon la revendication 7, dans lequel on 'utilise un solvant comprenant du toluène et du n-butanol dans un rapport volumique toluène : butanol inférieur à 1 : 2 pour extraire les constituants actifs désirés d'un extrait de feuilles de Ginkgo biloba.

9. Procédé selon la revendication 8, dans lequel le rapport volumique toluène : butanol est dans l'intervalle de 1 : 2 à 1 : 5.

10. Procédé selon la revendication 9, dans lequel le rapport volumique toluène : butanol est d'environ 1 : 4.

11. Procédé selon l'une quelconque des revendications précédentes, dans lequel on recueille une phase aqueuse comme produit de ces opérations d'extraction par les solvants, et on recueille des flavonoïdes dimères à partir de cette phase aqueuse.

12. Procédé selon l'une quelconque des revendications 1 à 10, dans lequel on met en contact une phase aqueuse récupérée au cours de l'opération de purification avec une résine d'absorption et on élue les flavonoïdes absorbées avec un solvant organique.

8

13. Procédé selon la revendication 12, dans lequel la résine d'absorption comprend un polymère aromatique.

14. Procédé selon les revendications 12 ou 13, dans lequel la résine d'absorption comprend une résine phénol-formaldéhyde contenant des groupes hydroxy.

15. Extrait de Ginkgo biloba, caractérisé par la présence de substances flavonoïdiques, de leurs dérivés glucosidatés, de ginkgolides, de bilobalide et de substances oligomères proanthocyanidiniques, et par l'absence pratique de substances lipophiles inactives qui peuvent être extraites par le n-hexane, le n-heptane ou un solvant comprenant un alcanol inférieur en $C_3$ à $C_6$ et une proportion prédominante d'un hydrocarbure aromatique en $C_6$ à $C_8$.

16. Extrait selon la revendication 15, qui peut être obtenu par un procédé tel que revendiqué dans l'une quelconque des revendications 1 à 14.

17. Extrait selon l'une quelconque des revendications 15 et 16 ayant pratiquement la composition suivante :

```
Teneur en glucosides de la ginkgoflavone    24 %
Indice procyanidolique                        9 %
Teneur en ginkgolides                       3,6 %
Teneur en bilobalide                        3,1 %
```